# EUROPÄISCHE PATENTSCHRIFT

(11) **EP 2 585 505 B1**
(45) Veröffentlichungstag und Bekanntmachung des Hinweises auf die Patenterteilung: **30.07.2014**
(21) Anmeldenummer: 11725776.6
(22) Anmeldetag: 20.06.2011
(51) Int. Cl.: C08G 18/10, C08G 18/48, A61L 15/26, A61L 15/46

(54) **VERFAHREN ZUR HERSTELLUNG VON HYDROPHILEN, ALIPHATISCHEN POLYURETHAN-SCHÄUMEN MIT NIEDRIGER ROHDICHTE**
METHOD FOR PRODUCING HYDROPHILIC, ALIPHATIC POLYURETHANE FOAMS HAVING LOW BULK DENSITY
PROCÉDÉ DE FABRICATION DE MOUSSES DE POLYURÉTHANE HYDROPHILES ALIPHATIQUES À FAIBLE DENSITÉ APPARENTE

(30) Priorität: 22.06.2010 EP 10166831
(43) Veröffentlichungstag der Anmeldung: 01.05.2013
(73) Patentinhaber: Bayer Intellectual Property GmbH, 40789 Monheim (DE)
(72) Erfinder: DÖRR, Sebastian, 40593 Düsseldorf (DE); NIESTEN, Meike, 51061 Köln (DE)
(74) Vertreter: BIP Patents
(86) Internationale Anmeldenummer: PCT/EP2011/060217
(87) Internationale Veröffentlichungsnummer: WO 2011/161048

(56) Entgegenhaltungen:
- EP-A1- 2 143 744

## Beschreibung

Die Erfindung betrifft ein Verfahren zur Herstellung von hydrophilen, aliphatischen Polyurethan-Schäumen mit niedriger Rohdichte. Weiterhin sind ein nach dem Verfahren erhältlicher hydrophiler, aliphatischer Polyurethan-Schaum und dessen Verwendung als Wundauflage, Inkontinenzprodukt oder als kosmetischer Artikel Gegenstände der Erfindung.

Aus der europäischen Patentanmeldung EP 2 143 744 A1 ist ein Verfahren bekannt, mit dessen Hilfe hydrophile, aliphatische Polyurethan-Schäume hergestellt werden können. Bei diesem Verfahren werden isocyanatfunktionelle Präpolymere mit C₈- bis C₂₂-Monocarbonsäuren oder deren Ammonium- oder Alkalisalzen oder C₁₂- bis C₄₄-Dicarbonsäuren oder deren Ammonium- oder Alkalisalzen und Wasser umgesetzt. Die Präpolymere sind durch Umsetzung von niedermolekularen, aliphatischen Diisocyanaten mit di- bis hexafunktionellen Polyalkylenoxiden erhältlich. Die Komponenten werden vermischt und in einen Becher gegeben, in dem dann die Schäumungsreaktion stattfindet. Dabei wird ein Polyurethan-Blockschaum erhalten. Dieser wird, wenn er beispielsweise als Wundauflage verwendet werden soll, auf die gewünschte Dicke von typischerweise 10 µ bis 5 cm zugeschnitten.

Bei dem Verfahren der EP 2 143 744 A1 werden ausschließlich Präpolymere eingesetzt, die einen Gehalt an niedermolekularen, aliphatischen Diisocyanaten von unter 1 Gewichtsprozent aufweisen. Auf diese Weise soll sicher gestellt werden, dass in dem fertigen Schaum keine extrahierbaren freien, niedermolekularen, aliphatischen Diisocyanate mehr enthalten sind, deren Freisetzung aus Wundauflagen als gesundheitlich bedenklich gilt.

Es war wünschenswert, über ein Verfahren zu verfügen, mit dessen Hilfe Schäume hergestellt werden können, die gegenüber den nach dem bekannten Verfahren erhältlichen Schäumen eine niedrigere Rohdichte aufweisen. Eine niedrige Rohdichte ist vorteilhaft, da bei gleichem Volumen weniger Material notwendig ist und durch den hohen Anteil an Poren und die damit verbundene kapillare Wirkung, ein schneller und guter Feuchtigkeitstransport möglich ist.

Aufgabe der vorliegenden Erfindung war daher ein einfaches Verfahren zur Herstellung von hydrophilen, aliphatischen Polyurethan-Schäumen mit niedriger Rohdichte und einem geringen Anteil an extrahierbaren freien Isocyanaten anzugeben.

Diese Aufgabe wird durch das Verfahren des Anspruchs 1 gelöst, bei dem
I) isocyanatfunktionelle Präpolymere A) durch Umsetzung von
   niedermolekularen, aliphatischen Diisocyanaten A1) einer Molmasse von 140 bis 278 g/mol mit
   di- bis hexafunktionellen Polyalkylenoxiden A2) einer OH-Zahl von 22,5 bis 112 mg KOH/g und einem Ethylenoxidanteil von 50 bis 100 mol%, bezogen auf die Gesamtmenge der enthaltenen Oxyalkylengruppen,
   hergestellt werden, wobei ein molarer Überschuss an niedermolekularen, aliphatischen Diisocyanaten A1) einsetzt wird,
II) der nicht abreagierte Überschuss an niedermolekularem, aliphatischen Diisocyanaten A1) zumindest teilweise entfernt wird,
III) ein Gemisch aus niedermolekularen, aliphatischen Diisocyanaten A1) einer Molmasse von 140 bis 278 g/mol mit den Präpolymeren A) hergestellt wird, in dem der Gehalt an freiem, niedermolekularen, aliphatischen Diisocyanat 1 bis 15 Gew.-% beträgt,
IV) das Gemisch des Schritts III) mit C8- bis C22-Monocarbonsäuren oder deren Ammonium- oder Alkalisalzen oder C12- bis C44-Dicarbonsäuren oder deren Ammonium- oder Alkalisalzen B) und Wasser C) vermischt, und
V) das Gemisch des Schritts IV) aufgeschäumt und ausgehärtet wird.

Überraschender Weise wurde gefunden, dass mit Hilfe des erfindungsgemäßen Verfahrens Polyurethan-Schäume erhalten werden können, die nicht nur eine im Vergleich zu Schäumen, die nach dem bekannten Verfahren hergestellten wurden, geringere Rohdichte haben, sondern darüber hinaus auch keinen höheren Anteil an extrahierbaren freien Isocyanaten aufweisen.

Gemäß einer bevorzugten Ausführungsform der Erfindung kann im Schritt III) ein Gemisch mit einem Gehalt an niedermolekularen, aliphatischen Diisocyanat A1) von 1,5 bis 10 und bevorzugt von 2,0 bis 8 Gew.% hergestellt werden. In diesem Fall werden Schäume mit einer besonders niedrigen Rohdichte erhalten.

Bevorzugt ist auch, wenn im Schritt II) der Überschuss an Diisocyanat A1) durch Dünnschichtdestillation entfernt wird, da diese Methode eine leicht und umfassende Entfernung ermöglicht.

Schäume mit besonders niedriger Rohdichte werden auch erhalten, wenn der NCO-Gehalt des Gemisches III), bestimmt nach DIN-EN ISO11909, 4 bis 10 Gew.-% beträgt.

Die in den Schritten I) und III) eingesetzten niedermolekularen, aliphatischen Diisocyanaten A1) haben jeweils eine Molmasse von 140 bis 278 g/mol. Bevorzugt handelt es sich jeweils um monomolekulare Verbindungen. Besonders bevorzugt sind die niedermolekularen, aliphatischen Diisocyanate A1) in Schritt I) und die niedermolekularen, aliphatischen Diisocyanate A1) in Schritt III) identische Verbindungen.

Beispiele für niedermolekulare, aliphatische Diisocyanate der Komponente A1) sind Hexamethylendiisocyanat (HDI), Isophorondiisocyanat (IPDI), Butylendiisocyanat (BDI), Bisisocyanatocyclohexylmethan (HMDI), 2,2,4-Trimethylhexamethylendiisocyanat, Bisisocyanatomethylcyclohexan, Bisisocyanatomethyltricyclodecan, Xylendiisocyanat, Tetramethylxylylendiisocyanat, Norbornandiisocyanat, Cyclohexandiisocyanat oder Diisocyanatododecan, wobei Hexamethylendiisocyanat, Isophorondiisocyanat, Butylendiisocyanat und Bis(isocyanatocyclohexyl)methan bevorzugt sind. Besonders bevorzugt sind Hexamethylendiisocyanat, Isophorondiisocyanat, Butylendiisocyanat und ganz besonders bevorzugt sind Hexamethylendiisocyanat und Isophorondiisocyanat.

Bevorzugt ist auch, wenn als Diisocyanat A1) ausschließlich Hexamethylendiisocyanat und Isophorondiisocyanat oder deren Mischungen untereinander eingesetzt werden.

Die Polyalkylenoxide A2) sind bevorzugt Copolymere aus Ethylenoxid und Propylenoxid, die auf Polyolen oder Aminen gestartet sind und einem Ethylenoxidgehalt, bezogen auf die Gesamtmenge der enthaltenen Oxyalkylengruppen, von 50 bis 100 mol%, bevorzugt von 60 bis 85 mol%, aufweisen. Geeignete Starter dieser Art sind Glycerin, Trimethylolpropan (TMP), Sorbit, Pentaerythrit, Triethanolamin, Ammoniak oder Ethylendiamin.

Die Polyalkylenoxide A2) besitzen typischerweise zahlenmittlere Molekulargewichte von 1000 bis 15000 g/mol, bevorzugt von 3000 bis 8500 g/mol.

Ferner können die Polyalkylenoxide A2) OH-Funktionalitäten von 2 bis 6, bevorzugt von 3 bis 6, besonders bevorzugt von 3 bis 4 besitzen.

In Weiterbildung der Erfindung ist vorgesehen, als Polyalkylenoxide A2) Copolymere aus Ethylenoxid und Propylenoxid mit einem Ethylenoxidgehalt, bezogen auf die Gesamtmenge der enthaltenen Oxyalkylengruppen, von 60 bis 85 mol%, gestartet auf Polyolen oder Aminen, einzusetzen.

Die Umsetzung der Diisocyanate A1) mit den Polyalkylenoxiden A2) kann in Gegenwart von Urethanisierungskatalysatoren wie Zinnverbindungen, Zinkverbindungen, Aminen, Guanidinen oder Amidinen, oder in Gegenwart von Allophanatisierungskatalysatoren wie Zinkverbindungen erfolgen.

Die Umsetzung kann typischerweise bei 25 bis 140°C, bevorzugt bei 60 bis 100°C erfolgen.

Als Komponente B) können Ammonium- und Alkalisalze von C₈- bis C₂₂-Monocarboxylaten oder deren freien Carbonsäuren oder Ammonium- und Alkalisalze von C₁₂- bis C₄₄-Dicarboxylaten oder deren freien Dicarbonsäuren, bevorzugt Kalium- oder Natriumsalze von C₈- bis C₂₂-Mono-carboxylaten oder von C₁₂- bis C₄₄-Dicarboxylaten und besonders bevorzugt Natriumsalze von Cgbis C₂₂-Monocarboxylaten eingesetzt werden.

Beispiele geeigneter Verbindungen der Komponente B) sind die Ammonium-, Na-, Li- oder K-Salze von Ethylhexansäure, Octansäure, Decansäure, Dodecansäure, Palmitinsäure, Stearinsäure, den Octadecensäuren, den Octadecadiensäuren, den Octadecatriensäuren, Isostearinsäure, Erucasäure, Abietinsäure und ihren Hydrierungsprodukten. Beispiele für C₁₂- bis C₄₄-Dicarbonsäuren bzw. die daraus abgeleiteten Ammonium- und Alkalisalze sind Dodecandisäure, Dodecenyl-, Tetradecenyl-, Hexadecenyl- und Octadecenyl-Bernsteinsäure, C₃₆- und C₄₄-Dimerfettsäuren und ihre Hydrierungsprodukte sowie die entsprechenden Ammonium-, Na-, Li- oder K-Salze dieser Dicarbonsäuren.

Das einzusetzende Wasser C) kann als solches, als Kristallwasser eines Salzes, als Lösung in einem dipolar-aprotischen Lösungsmittel oder auch als Emulsion eingesetzt werden. Bevorzugt wird das Wasser als solches oder in einem dipolar-aprotischen Lösungsmittel verwendet.

Es ist weiterhin möglich, dass das Gemisch des Schritts IV) gegebenenfalls Katalysatoren D), Tenside E) Alkohole F) und / oder Treibmittel G) enthält.

Als Katalysatoren D) können insbesondere einzeln oder in Kombination Metallsalze, Amine, Amidine und Guanidine verwendet werden.

Zur Verbesserung der Schaumbildung, Schaumstabilität oder der Eigenschaften des resultierenden Polyurethan-Schaums können die Verbindungen der Komponente E) eingesetzt werden, wobei solche Additive grundsätzlich alle an sich bekannten anionischen, kationischen, amphoteren und nichtionischen Tenside sowie Mischungen hieraus sein können. Bevorzugt werden Alkylpolyglycoside, EO/PO-Blockcopolymere, Alkyl- oder Arylalkoxylate, Siloxanalkoxylate, Ester der Sulfobernsteinsäure und/oder eingesetzt. Besonders bevorzugt werden EO/PO-Blockcopolymere eingesetzt. Bevorzugt werden allein die EO/PO-Blockcopolymere als Komponente E) eingesetzt.

Zudem können zur Verbesserung der Schaumeigenschaften des resultierenden Polyurethan-Schaums Verbindungen der Komponente F) eingesetzt werden. Hierbei handelt es sich grundsätzlich um alle dem Fachmann an sich bekannten ein- und mehrwertigen Alkohole sowie Mischungen hieraus. Dies sind ein- oder mehrwertige Alkohole oder Polyole, wie Ethanol, Propanol, Butanol, Decanol, Tridecanol, Hexadecanol, Ethylenglykol, Neopentylglykol, Butandiol, Hexandiol, Decandiol, Trimethylolpropan, Glycerin, Pentaerythrit, monofunktionelle Polyetheralkohole und Polyesteralkohole, Polyetherdiole und Polyesterdiole.

Das Aufschäumen kann grundsätzlich durch das bei der Reaktion der Isocyanatgruppen mit Wasser gebildete Kohlendioxid erfolgen, die Verwendung von weiteren Treibmitteln G) ist jedoch ebenfalls möglich. So können prinzipiell auch Treibmittel aus der Klasse der Kohlenwasserstoffe wie C₃-C₆-Alkane, z.B. Butane, *n*-Pentan, *iso*-Pentan, *cyclo*-Pentan, Hexane o.ä. oder halogenierte Kohlenwasserstoffe wie Dichlormethan, Dichlormonofluormethan, Chlordifluorethane, 1,1-Dichlor-2,2,2-Trifluorethan, 2,2-Dichlor-2-fluorethan, insbesondere chlorfreie Fluorkohlenwasserstoffe wie Difluormethan, Trifluormethan, Difluorethan, 1,1,1,2-Tetrafluorethan, Tetrafluorethan (R 134 oder R 134a), 1,1,1,3,3-Pentafluorpropan (R 245 fa), 1,1,1,3,3,3-Hexafluorpropan (R 256), 1,1,1,3,3-Pentafluorbutan (R 365 mfc), Heptafluorpropan oder auch Schwefelhexafluorid verwendet werden. Auch Gemische dieser Treibmittel sind verwendbar.

Gemäß einer bevorzugten Ausführungsform der Erfindung ist vorgesehen, dass die Komponenten A) bis F) in den folgenden Mengen eingesetzt werden:
100 Gewichtsteile des Gemisches des Schritts III)
0,1 bis 5 Gewichtteile C8- bis C22-Monocarbonsäuren oder deren Ammonium- oder Alkalisalze oder C12- bis C44-Dicarbonsäuren oder deren Ammonium- oder Alkalisalze B)
1 bis 200 Gewichtsteile Wasser C)
0 bis 1 Gewichtteile Katalysatoren D)
0 bis 10 Gewichtsteile Tenside E)
0 bis 20 Gewichtsteile Alkohole F)

Besonders bevorzugt werden die Komponenten A) bis F) in folgenden Mengen eingesetzt:
100 Gewichtsteile des Gemisches des Schritts I)
0,1 bis 5 Gewichtteile an C8- bis C22-Monocarbonsäuren oder deren Ammonium- oder Alkalisalze oder C12- bis C44-Dicarbonsäuren oder deren Ammonium- oder Alkalisalze B)
2 bis 100 Gewichtsteile Wasser C)
0 bis 1 Gewichtteile an Katalysatoren D)
0 bis 10 Gewichtsteile Tenside E)
0 bis 20 Gewichtsteile Alkohole F).

Ganz besonders bevorzugt werden die Komponenten A) bis F) in folgenden Mengen eingesetzt:
100 Gewichtsteile des Gemisches des Schritts I)
0,1 bis 5 Gewichtteile an C8- bis C22-Monocarbonsäuren oder deren Ammonium- oder Alkalisalze oder C12- bis C44-Dicarbonsäuren oder deren Ammonium- oder Alkalisalze B)
3 bis 50 Gewichtsteile Wasser C)
0 bis 1 Gewichtteile an Katalysatoren D)
0 bis 10 Gewichtsteile Tenside E)
0 bis 20 Gewichtsteile Alkohole F).

Beim Durchmischen der Komponenten und/oder Gemische und während der Schäumungsreaktion kann bei Temperaturen von 0 bis 100°C gearbeitet werden, bevorzugt bei 15 bis 70°C, ganz besonders bevorzugt bei 20 bis 50°C.

Nach dem Mischen der Komponente kann das Gemisch auf ein flächiges Substrat als Schicht mit konstanter Dicke aufgebracht werden. Geeignete Substrate sind zum Beispiel Trennfolien oder Trennpapiere, die perforiert sein können.

Vorzugsweise kann das Gemisch auf das Substrat aufgerakelt werden. Dazu kann das Gemisch in einem Rakelkasten gegossen und in einer bestimmten Dicke horizontal in flächigen Matten auf ein geeignetes Substrat wie z.B. eine Trennfolie oder einen Trennpapier gerakelt werden.

Die Spalthöhe des Rakels liegt dabei im Allgemeinen im Bereich von 0,2 bis 20 mm, bevorzugt von 0,2 bis 5 und ganz besonders bevorzugt von 0,2 bis 2 mm. Die Filmbreite des zu verwendenden Rakels kann dem jeweiligen Verwendungszweck angepasst werden. Beispiele sind Filmbreiten zwischen 10 und 5000 mm, bevorzug zwischen 10 und 4000 mm.

Als Rakel können alle bekannten Typen wie beispielsweise Luftrakel, Walzenrakel, Streichrakel, Kastenrakel, Messerrakel oder Magnetrakel eingesetzt werden. Als Material für die Rakel kommen alle üblichen Materialien in Betracht, z.B. Metalle wie Edelstahl oder Kunststoffe. Auch ein Verbund mehrerer Materialien zur Herstellung des Rakels ist möglich. Es können sowohl Handrakel als auch Maschinenrakel eingesetzt werden, bevorzugt ist die Verwendung von Maschinenrakeln, eingebunden in geeigneten Beschichtungsanlagen. Auch die Applikation zwischen Walzen ist möglich.

Auf die Schicht des Gemisches kann direkt nach dem Auftragen ein perforiertes Trennelement flächig aufgelegt, so dass es die dem Substrat abgewandte Oberfläche der Schicht bedeckt.

Unter perforiert wird vorliegend ein Trennelement verstanden, dass eine Vielzahl von, das Trennelement von der Auflagefläche aus durchsetzenden Aussparungen aufweist.

Die Aussparungen haben bevorzugt einen kreisförmigen Durchmesser.

Bevorzugt ist auch, wenn die Aussparungen gleichmäßig über das Trennelement verteilt sind.

Die Aussparungen können bevorzugt einen Durchmesser von 20 bis 300 µm haben. In diesem Fall erhält man Schäume, bei denen keine Erhebungen in Form der Aussparrungen des Trennelements auf der Schaumoberfläche sichtbar sind. Die Schäume haben eine glatte Oberfläche, was insbesondere bei deren Verwendung als Wundauflage vorteilhaft ist, da diese möglichst flächig am Körper anliegen sollen.

Der Abstand zwischen zwei benachbarten Aussparungen liegt vorzugsweise zwischen 0,1 bis 5 mm, weiter bevorzugt zwischen 0,5 bis 3 mm und ganz besonders bevorzugt zwischen 0,8 und 2,5 mm.

Bei dem perforierten Trennelement kann es sich beispielsweise um ein perforiertes Trennpapier oder eine perforierte Trennfolie handeln. Das Trennpapier kann z.B. silikonisiertes Papier, Polyolefinbeschichtetes Papier oder Fluorcarbon-beschichtetes Papier sein. Ebenso kann die Trennfolie aus Silkon, Polyolefinen und / oder Fluorcarbon bestehen, bzw. mit derartigen Materialien beschichtet sein.

Bevorzugt kann nach dem Aufbringen das Trennelement insbesondere noch mit einem definierten Druck auf die Schicht aus dem Gemisch des Schritts IV) gepresst werden.

Um die Aushärtung des Polyurethan-Schaums nach Beendigung der Expansion zu beschleunigen, kann dieser erwärmt werden. Vorzugsweise kann der Polyurethan-Schaum auf eine Temperatur von 40 bis 140 °C, weiter bevorzugt von 60 bis 120 °C und besonders bevorzugt von 60 bis 110 °C erwärmt werden.

Besonders bevorzugt ist auch ein Verfahren, bei dem aus dem erfindungsgemäßen Polyurethan-Schaum eine Wundauflage hergestellt wird.

Weitere Gegenstände der Erfindung sind ein nach dem erfindungsgemäßen Verfahren herstellbarer Polyurethan-Schaum.

Die erhaltenen Polyurethan-Schäume weisen eine poröse, zumindest teilweise offenzellige Struktur mit miteinander kommunizierenden Zellen auf.

Die Polyurethan-Schäume können mit weiteren Materialien verklebt, laminiert oder beschichtet werden, beispielsweise auf Basis von Hydrogelen, (semi-) permeablen Folien, Schaumfolien, Beschichtungen, Hydrokolloiden oder anderen Schäumen.

Die erfindungsgemäßen Polyurethan-Schäume sind besonders geeignet zur Herstellung von Wundauflagen. Dabei können die Polyurethan-Schäume in direktem oder indirektem Kontakt mit der Wunde sein. Bevorzugt werden die Polyurethan-Schäume jedoch in direktem Kontakt mit der Wunde eingesetzt, um beispielsweise eine optimale Absorption von Wundflüssigkeit zu gewährleisten. Die Polyurethan-Schäume zeigen keine Zelltoxizität (Bestimmung nach ISO 10993-5 und ISO 10993-12).

Die Polyurethan-Schäume, welche als Wundauflage eingesetzt werden, können zusätzlich noch in einem weiteren Verfahrensschritt sterilisiert werden. Zur Sterilisation kommen die dem Fachmann an sich bekannten Verfahren zum Einsatz, bei denen eine Sterilisation durch thermische Behandlung, chemische Stoffe wie Ethylenoxid oder Bestrahlung, beispielsweise durch Gammastrahlung, erfolgt. Die Bestrahlung kann dabei gegebenenfalls unter Schutzgasatmosphäre erfolgen. Die erfmdungsgemäßen Polyurethan-Schäume haben dabei den großen Vorteil, dass sie sich bei Bestrahlung, insbesondere bei Bestrahlung mit Gammastrahlen, nicht verfärben.

Ebenfalls möglich ist die Zugabe, Einarbeitung oder Beschichtung von bzw. mit antimikrobiellen oder biologischen Wirkstoffen, welche sich beispielsweise in Bezug auf die Wundheilung und die Vermeidung von Keimbelastungen positiv auswirken.

Gegenstand der Erfindung ist schließlich auch ein erfindungsgemäßer hydrophiler, aliphatischer Polyurethan-Schaum zur Verwendung als Wundauflage. Inkontinenzprodukt oder kosmetischer Artikel.

### Beispiele

Sofern nicht abweichend gekennzeichnet, beziehen sich alle Prozentangaben auf das Gewicht. Die Bestimmung der Festkörpergehalte erfolgte nach DIN-EN ISO 3251. Die Bestimmung der Viskositäten erfolgte bei 23 °C und wurde nach DIN 53019 durchgeführt. Die NCO-Gehalte wurden volumetrisch gemäß DIN-EN ISO11909 bestimmt.

### Bestimmung der Extraktmenge:

**48 h:** 10 g des Schaums wurden für 48 Stunden in 300 ml vollentsalztes Wasser bei 37°C eingelegt und durch Titration des chemischen Sauerstoffbedarfs gemäß DIN EN 1484 die Extraktmenge bestimmt.

**7 Tage:** 4,7 g des Schaums wurden für 7 Tage in 220 ml vollentsalztes Wasser bei 37°C eingelegt und durch Titration des chemischen Sauerstoffbedarfs gemäß DIN EN 1484 die Extraktmenge bestimmt.

### Bestimmung der Rohdichte

Zur Bestimmung der Rohdichte wurde zunächst ein 10x10x5 cm großes Stück des jeweiligen Schaums gewogen. Anschließend wurde die Rohdichte berechnet, indem die Masse des Schaums durch sein Volumen geteilt wurde.

### Verwendete Substanzen und Abkürzungen:

| | |
|---|---|
| Carboxylat 1: | 5% Natriumoleat in Wasser |
| Desmodur^{®} N 3400: | Aliphatisches Polyisocyanat (HDI-Uretdion), NCO-Gehalt 21,8% |
| DBU | 1,8-Diazabi-cyclo[5.4.0]undecen-7 |

### Beispiel1: Herstellung des Polyurethan-Präpolymers 1, Dünnschichtverfahren, Komponente zur definierten Abmischung mit monomerem Diisocyanat

Zu einem Gemisch aus 1000g Hexamethylendiisocyanat (HDI) und 1g Benzoylchlorid wurde bei 80°C innerhalb von 3h 1000g eines Polyalkylenoxids mit einer Molmasse von 4680 g/mol gestartet auf Glycerin, einem Ethylenoxidgewichtsanteil von 72 % und Propylenoxidgewichtsanteil von 28 %, das zuvor bei 100°C während 2h bei einem Druck von 10 mbar getrocknet wurde, zugetropft und für 12h nachgerührt. Das überschüssige HDI wurde durch Dünnschichtdestillation bei 130°C und 0,1 mbar entfernt, wobei die nicht flüchtigen Bestandteile mit 1 g Chlorpropionsäure stabilisiert wurden. Man erhielt ein Präpolymer mit einem NCO-Gehalt von 2,8% und einer Viskosität von 3500 mPas.

### Beispiele 2 und 3, Vergleichbeispiele 1 und 2: Herstellung von Schäumen

Die Isocyanat-Komponenten wurden 15 Sekunden mit einer Rührerdrehzahl von 1200 Upm homogenisiert. Dann wurden die weiteren Komponenten eingewogen und weitere 10 Sekunden verrührt. Schließlich wurde die Mischung in eine Papierform von 15x15x15 (Breite x Höhe x Länge) gegossen. Als Oligomer wurde, falls verwendet, dabei jeweils Desmodur® N 3400 eingesetzt; als Carboxylat eine 5 Gew.-%ige Lösung von Natriumoleat in Wasser. Darüber hinaus zugesetztes Wasser ist extra angegeben.

| | **2** | **3** | **Vergleichsbeispiel 1** | **Vergleichsbeispiel 2** |
|---|---|---|---|---|
| Prepolymer DSB 3227 | 75 | 75 | 75 | 75 |
| HDI | 5,3 | 8,1 | | |
| Oligomer (N3400) | 0 | 0 | | 8,33 |
| NCO-Gehalt Mischung (%) | 4,6 | 6 | 2,6 | 4,6 |
| Wasser | 3,35 | 3,35 | 3,35 | 3,35 |
| DBU | 0,05 | 0,05 | 0,05 | 0,05 |
| Natrium-Oleat (Baustein E) als 5 Gew.-%ige Lösung in Wasser | 8,15 | 8,15 | 8,15 | 8,15 |
| Rohdichte [g/1000 cm³] | 71 | 83 | 199 | 109 |
| Extrakt EN 1484 wt% | | | | |
| 48 h | 0,11 | 0,08 | Nicht bestimmt | 0,11 |
| 7 t | 0,13 | 0,11 | | 0,15 |

Die Beispiele 2 und 3 zeigen, dass auf Basis von Präpolymeren, die freies niedermolekulares, aliphatisches Diisocyanat enthalten, Schäume mit einer Rohdichte von kleiner als 100 g/l hergestellt werden können. Die Extraktmenge dieser Schäume ist dabei nicht größer als die des Schaums aus dem Vergleichsbeispiel 2, der auf einem gedünnschichteten Präpolymer basiert, dass frei von monomerem Diisocyanat ist.

Das Vergleichsbeispiel 1 zeigt, dass Schäume, die aus gedünnschichteten Präpolymer ohne zusätzliche Zugabe von einem freien Diisocyanate hergestellt werden, eine deutlich höhere Rohdichte haben.

## Patentansprüche

1. Verfahren zur Herstellung von hydrophilen, aliphatischen Polyurethan-Schäumen, bei dem
I) isocyanatfunktionelle Präpolymere A) durch Umsetzung von
niedermolekularen, aliphatischen Diisocyanaten A1) einer Molmasse von 140 bis 278 g/mol mit
di- bis hexafunktionellen Polyalkylenoxiden A2) einer OH-Zahl von 22,5 bis 112 mg KOH/g und einem Ethylenoxidanteil von 50 bis 100 mol%, bezogen auf die Gesamtmenge der enthaltenen Oxyalkylengruppen,
hergestellt werden, wobei ein molarer Überschuss an niedermolekularen, aliphatischen Diisocyanaten A1) einsetzt wird,
II) der nicht abreagierte Überschuss an niedermolekularem, aliphatischen Diisocyanaten A1) zumindest teilweise entfernt wird,
III) ein Gemisch aus niedermolekularen, aliphatischen Diisocyanaten A1) mit den Präpolymeren A) hergestellt wird, in dem der Gehalt an freiem, niedermolekularen, aliphatischen Diisocyanat 1 bis 15 Gew.-% beträgt,
IV) das Gemisch des Schritts III) mit C8- bis C22-Monocarbonsäuren oder deren Ammonium- oder Alkalisalzen oder C12- bis C44-Dicarbonsäuren oder deren Ammonium- oder Alkalisalzen B) und Wasser C) vermischt, und
V) das Gemisch des Schritts IV) aufgeschäumt und ausgehärtet wird.

2. Verfahren nach Anspruch 1, **dadurch gekennzeichnet, dass** im Schritt III) ein Gemisch mit einem Gehalt an niedermolekularen, aliphatischen Diisocyanat A1) von 1,5 bis 10 und bevorzugt von 2,0 bis 8 Gew.-% hergestellt wird.

3. Verfahren nach einem der Ansprüche 1 oder 2, **dadurch gekennzeichnet, dass** im Schritt II) der Überschuss an Diisocyanat A1) durch Dünnschichtdestillation entfernt wird.

4. Verfahren nach einem der Ansprüche 1 bis 3, **dadurch gekennzeichnet, dass** der NCO-Gehalt des Gemisches III), bestimmt nach DIN-EN ISO 11909, 4 bis 10 Gew.% beträgt.

5. Verfahren nach einem der Ansprüche 1 bis 4, **dadurch gekennzeichnet, dass** als Diisocyanat A1) ausschließlich Hexamethylendiisocyanat, Isophorondiisocyanat oder deren Mischungen untereinander eingesetzt werden.

6. Verfahren nach einem der Ansprüche 1 bis 5, **dadurch gekennzeichnet, dass** als Polyalkylenoxide A2) Copolymere aus Ethylenoxid und Propylenoxid mit einem Ethylenoxidgehalt, bezogen auf die Gesamtmenge der enthaltenen Oxyalkylengruppen, von 60 bis 85 mol%, gestartet auf Polyolen oder Aminen eingesetzt werden.

7. Verfahren nach einem der Ansprüche 1 bis 6, **dadurch gekennzeichnet, dass** die Polyalkylenoxide A2) zahlenmittlere Molekulargewichte von 3000 bis 8500 g/mol aufweisen.

8. Verfahren nach einem der Ansprüche 1 bis 7, **dadurch gekennzeichnet, dass** die Polyalkylenoxide A2) OH-Funktionalitäten von 3 bis 4 aufweisen.

9. Verfahren nach einem der Ansprüche 1 bis 8, **dadurch gekennzeichnet, dass** das Gemisch des Schritts IV) gegebenenfalls Katalysatoren D), Tenside E), Alkohole F) und / oder Treibmittel G) enthält.

10. Verfahren nach Anspruch 9, **dadurch gekennzeichnet, dass** als Katalysatoren F) einzeln oder in Kombination Metallsalze, Amine, Amidine und Guanidine verwendet werden.

11. Verfahren einem der Ansprüche 9 oder 10 **dadurch gekennzeichnet, dass** die Komponenten A) bis F) in den folgenden Mengen eingesetzt werden:
100 Gewichtsteile des Gemisches des Schritts III)
0,1 bis 5 Gewichtteile C8- bis C22-Monocarbonsäuren oder deren Ammonium- oder Alkalisalze oder C12- bis C44-Dicarbonsäuren oder deren Ammonium- oder Alkalisalze B)
1 bis 200 Gewichtsteile Wasser C)
0 bis 1 Gewichtteile Katalysatoren D)
0 bis 10 Gewichtsteile Tenside E)
0 bis 20 Gewichtsteile Alkohole F).

12. Verfahren nach einem der Ansprüche 1 bis 11, **dadurch gekennzeichnet, dass** der Polyurethan-Schaum nach Beendigung der Aufschäumung zur Beschleunigung der Aushärtung erwärmt wird, bevorzugt auf eine Temperatur von 40 bis 140 °C, weiter bevorzugt von 60 bis 120 °C und besonders bevorzugt von 60 bis 110 °C.

13. Verfahren nach einem der Ansprüche 1 bis 12, **dadurch gekennzeichnet, dass** aus dem Polyurethan-Schaum eine Wundauflage hergestellt wird.

14. Hydrophiler, aliphatischer Polyurethan-Schaum, erhältlich nach einem Verfahren gemäß einem der Ansprüche 1 bis 13.

15. Hydrophiler, aliphatischer Polyurethan-Schaum nach Anspruch 14 zur Verwendung als Wundauflage. Inkontinenzprodukt oder kosmetischer Artikel.

16. Verwendung eines hydrophilen, aliphatischen Polyurethan-Schaums nach Anspruch 14 zur Herstellung eines Mittels zur Behandlung von Wunden.

## Claims

1. Method for producing hydrophilic aliphatic polyurethane foams which comprises
I) preparing isocyanate-functional prepolymers A) by reaction of
low molecular weight aliphatic diisocyanates A1) having a molar mass of 140 to 278 g/mol with
di- to hexafunctional polyalkylene oxides A2) having an OH number of 22.5 to 112 mg KOH/g and an ethylene oxide fraction of 50 to 100 mol%, based on the total amount of oxyalkylene groups present,
using a molar excess of low molecular weight aliphatic diisocyanates A1),
II) at least partly removing the unreacted excess of low molecular weight aliphatic diisocyanates A1),
III) preparing a mixture of low molecular weight aliphatic diisocyanates A1) having a molar mass of 140 to 278 g/mol with the prepolymers A) such that the free low molecular weight aliphatic diisocyanate content of said mixture is 1 to 15 wt%,
IV) mixing the mixture of step III) with C8- to C22-monocarboxylic acids or their ammonium or alkali metal salts or C12- to C44-dicarboxylic acids or their ammonium or alkali metal salts B) and water C), and
V) foaming and curing the mixture of step IV).

2. Method according to Claim 1, **characterized in that** in step III) a mixture having a low molecular weight aliphatic diisocyanate A1) content of 1.5 to 10 and preferably of 2.0 to 8 wt% is prepared.

3. Method according to either Claim 1 or 2, **characterized in that** in step II) the excess of diisocyanate A1) is removed by thin film distillation.

4. Method according to any of Claims 1 to 3, **characterized in that** the NCO content of the mixture III), determined to DIN-EN ISO 11909, is 4 to 10 wt%.

5. Method according to any of Claims 1 to 4, **characterized in that** the diisocyanate A1) used is exclusively hexamethylene diisocyanate, isophorone diisocyanate or mixtures thereof.

6. Method according to any of Claims 1 to 5, **characterized in that** the polyalkylene oxides A2) used are copolymers of ethylene oxide and propylene oxide having an ethylene oxide content, based on the total amount of oxyalkylene groups present, of 60 to 85 mol% and started on polyols or amines.

7. Method according to any of Claims 1 to 6, **characterized in that** the polyalkylene oxides A2) have number-average molecular weights of 3000 to 8500 g/mol.

8. Method according to any of Claims 1 to 7, **characterized in that** the polyalkylene oxides A2) have OH functionalities of 3 to 4.

9. Method according to any of Claims 1 to 8, **characterized in that** the mixture of step IV) optionally contains catalysts D), surfactants E), alcohols F) and/or blowing agents G).

10. Method according to Claim 9, **characterized in that** catalysts F) are metal salts, amines, amidines and guanidines used alone or in combination.

11. Method according to either Claim 9 or 10, **characterized in that** the components A) to F) are used in the following amounts:
100 parts by weight of the mixture of step III),
0.1 to 5 parts by weight of C8- to C22-monocarboxylic acids or their ammonium or alkali metal salts or C12- to C44-dicarboxylic acids or their ammonium or alkali metal salts B),
1 to 200 parts by weight of water C),
0 to 1 part by weight of catalysts D),
0 to 10 parts by weight of surfactants E),
0 to 20 parts by weight of alcohols F).

12. Method according to any of Claims 1 to 11, **characterized in that** the polyurethane foam on completion of foaming is heated, preferably to a temperature of 40 to 140°C, more preferably of 60 to 120°C and even more preferably of 60 to 110°C, to speed curing.

13. Method according to any of Claims 1 to 12, **characterized in that** a wound dressing is produced from the polyurethane foam.

14. Hydrophilic aliphatic polyurethane foam obtainable according to a method according to any of Claims 1 to 13.

15. Hydrophilic aliphatic polyurethane foam according to Claim 14 for use as wound dressing, incontinence product or cosmetic article.

16. Use of a hydrophilic aliphatic polyurethane foam according to Claim 14 for preparing a composition for treating wounds.

## Revendications

1. Procédé pour la production de mousses de polyuréthane aliphatique, hydrophiles, dans lequel
I) on prépare des prépolymères A) à fonction isocyanate par mise en réaction
de diisocyanates aliphatiques A1), de faible masse moléculaire, ayant une masse moléculaire de 140 à 278 g/mole, avec
des polyoxyalkylènes di- à hexa-fonctionnels A2) ayant un indice de groupes OH de 22,5 à 112 mg de KOH/g et une proportion d'oxyde d'éthylène de 50 à 100 % en moles, par rapport à la quantité totale des groupes oxyalkylène contenus,
en utilisant un excès molaire de diisocyanates aliphatiques A1), de faible masse moléculaire,
II) on élimine au moins en partie l'excès n'ayant pas réagi de diisocyanates aliphatiques A1), de faible masse moléculaire,
III) on prépare un mélange de diisocyanates aliphatiques A1), de faible masse moléculaire, ayant une masse moléculaire de 140 à 278 g/mole, avec les prépolymères A), dans lequel la teneur en diisocyanate aliphatique, de faible masse moléculaire, libre, vaut de 1 à 15 % en poids,
IV) on mélange le mélange de l'étape III) avec des acides monocarboxyliques en C₈-C₂₂ ou leurs sels de métaux alcalins ou d'ammonium ou des acides dicarboxyliques en C₁₂-C₄₄ ou leurs sels de métaux alcalins ou d'ammonium B) et de l'eau C), et
V) le mélange de l'étape IV) est transformé en mousse et durci.

2. Procédé selon la revendication 1, **caractérisé en ce que** dans l'étape III) on prépare un mélange ayant une teneur en diisocyanate aliphatique A1), de faible masse moléculaire, de 1,5 à 10 et de préférence de 2,0 à 8 % en poids.

3. Procédé selon l'une quelconque des revendications 1 et 2, **caractérisé en ce que** dans l'étape II) on élimine l'excès de diisocyanate A1) par distillation en couche mince.

4. Procédé selon l'une quelconque des revendications 1 à 3, **caractérisé en ce que** la teneur en NCO du mélange III), déterminée selon DIN-EN ISO 11909, vaut de 4 à 10 % en poids.

5. Procédé selon l'une quelconque des revendications 1 à 4, **caractérisé en ce qu'**on utilise comme diisocyanate A1) exclusivement l'hexaméthylène-diisocyanate, l'isophoronediisocyanate ou des mélanges de ceux-ci entre eux.

6. Procédé selon l'une quelconque des revendications 1 à 5, **caractérisé en ce qu'**on utilise comme polyoxyalkylènes A2) des copolymères d'oxyde d'éthylène et d'oxyde de propylène ayant une teneur en oxydes d'éthylène, par rapport à la quantité totale des groupes oxyalkylène contenus, de 60 à 85 % en moles, obtenus à partir de polyols ou d'amines.

7. Procédé selon l'une quelconque des revendications 1 à 6, **caractérisé en ce que** les polyoxyalkylènes A2) présentent des masses moléculaires moyennes en nombre de 3 000 à 8 500 g/mole.

8. Procédé selon l'une quelconque des revendications 1 à 7, **caractérisé en ce que** les polyoxyalkylènes A2) présentent des fonctionnalités OH de 3 à 4.

9. Procédé selon l'une quelconque des revendications 1 à 8, **caractérisé en ce que** le mélange de l'étape IV) contient éventuellement des catalyseurs D), des tensioactifs E), des alcools F) et/ou des agents porogènes G).

10. Procédé selon la revendication 9, **caractérisé en ce qu'**on utilise comme catalyseurs F) des sels métalliques, des amines, des amidines et des guanidines, individuellement ou en association.

11. Procédé selon l'une quelconque des revendications 9 et 10, **caractérisé en ce qu'**on utilise les composants A) à F) en les quantités suivantes :
100 parties en poids du mélange de l'étape III)
0,1 à 5 parties en poids d'acides monocarboxyliques en C₈-C₂₂ ou de leurs sels de métaux alcalins ou d'ammonium ou d'acides dicarboxyliques en C₁₂-C₄₄ ou de leurs sels de métaux alcalins ou d'ammonium B)
1 à 200 parties en poids d'eau C)
0 à 1 partie en poids de catalyseurs D)
0 à 10 parties en poids de tensioactifs E)
0 à 20 parties en poids d'alcools F).

12. Procédé selon l'une quelconque des revendications 1 à 11, **caractérisé en ce qu'**une fois terminé le moussage on chauffe la mousse de polyuréthane, afin d'accélérer le durcissement, de préférence jusqu'à une température de 40 à 140 °C, encore mieux de 60 à 120 °C et de façon particulièrement préférée de 60 à 110 °C.

13. Procédé selon l'une quelconque des revendications 1 à 12, **caractérisé en ce qu'**à partir de la mousse de polyuréthane on fabrique un pansement.

14. Mousse de polyuréthane aliphatique, hydrophile, pouvant être obtenue conformément à un procédé selon l'une quelconque des revendications 1 à 13.

15. Mousse de polyuréthane aliphatique, hydrophile, selon la revendication 14, destinée à l'utilisation en tant que pansement, produit pour incontinence ou article cosmétique.

16. Utilisation d'une mousse de polyuréthane aliphatique, hydrophile, selon la revendication 14, pour la fabrication d'un produit destiné au traitement de plaies.
